# EUROPÄISCHE PATENTSCHRIFT

(11) **EP 3 698 474 B1**
(45) Veröffentlichungstag und Bekanntmachung des Hinweises auf die Patenterteilung: **03.11.2021**
(21) Anmeldenummer: 18786328.7
(22) Anmeldetag: 12.10.2018
(51) Int. Cl.: H03G 5/02

(54) **VERFAHREN ZUR EINSTELLUNG VON PARAMETERN ZUR INDIVIDUELLEN ANPASSUNG EINES AUDIOSIGNALS**
METHOD FOR THE ADJUSTMENT OF PARAMETERS FOR THE INDIVIDUAL MODIFICATION OF AN AUDIO SIGNAL
PROCÉDÉ DE RÉGLAGE DES PARAMÈTRES PERMETTANT D'AJUSTER INDIVIDUELLEMENT UN SIGNAL AUDIO

(30) Priorität: 16.10.2017 DE 102017218483
(43) Veröffentlichungstag der Anmeldung: 26.08.2020
(73) Patentinhaber: Fraunhofer-Gesellschaft zur Förderung der angewandten Forschung e.V., 80686 München (DE)
(72) Erfinder: OETTING, Dirk, 26129 Oldenburg (DE); BRUNS, Tobias, 26129 Oldenburg (DE); RENNIES-HOCHMUTH, Jan, 26129 Oldenburg (DE)
(74) Vertreter: Zimmermann, Tankred Klaus
(86) Internationale Anmeldenummer: PCT/EP2018/077951
(87) Internationale Veröffentlichungsnummer: WO 2019/076773

(56) Entgegenhaltungen:
- DE-B3-102006 046 699
- US-A1- 2004 234 089
- US-B2- 8 165 327

## Beschreibung

Ausführungsbeispiele der vorliegenden Erfindung beziehen sich auf ein Verfahren sowie eine Vorrichtung zur Einstellung von Parametern, mittels welchen eine individuelle Anpassung eines Audiosignals erfolgen kann. Ein weiteres Ausführungsbeispiel bezieht sich auf ein Computerprogramm. Bevorzugte Ausführungsbeispiele beziehen sich auf die individuelle Klanganpassung unter Berücksichtigung der effektiven Hörschwelle.

Um Musiksignale an das individuelle Gehör anzupassen, ist eine verbreitete Variante in heutigen Audiogeräte einen Equalizer anzupassen. Mit einem Equalizer kann eine frequenzabhängige Verstärkung auf das Musiksignal angewendet werden, bevor es abgespielt wird. Die Bedienung eines Equalizers ist für den Laien aber häufig kompliziert, da nur einzelne Frequenzbänder zurzeit geändert werden können. Deshalb sind in der Vergangenheit Methoden entwickelt worden, um in einem vereinfachten oder geführten Verfahren den Klang an das Gehör anzupassen. Dabei sind verschiedene Aspekte zu berücksichtigen, die einen Einfluss auf den wahrgenommenen Klang haben: der akustische Pfad vom Lautsprecher/Kopfhörer zum Ohr, die technischen Grenzen für den maximalen akustischen Pegel, das Hintergrundgeräusch, eine mögliche Einschränkung des Hörvermögens sowie der spektrale Gehalt des Musikstücks, für das die Anpassung erfolgen soll. Zur Kompensation des Hörvermögens verwenden einige Methoden Messverfahren, um die individuelle Hörschwelle zu ermitteln. Neuere Studien zeigen, dass auf Grund der Hörschwelle nicht auf die präferierte Klangeinstellung beim Musikhören geschlossen werden kann, d.h., Menschen mit derselben frequenzabhängigen Hörschwelle können sehr unterschiedliche Klangpräferenzen haben. Das Hören von Musik bei normaler Lautstärke (also oberhalb der Hörschwelle) wird nur sehr begrenzt durch die absolute Hörschwelle bestimmt. Ferner unterscheiden sich Klangpräferenzen zwischen unterschiedlichen Musikstücken, so dass es nicht "die" optimale Equalizer-Einstellung für verschiedene Musikstücke gibt.

Einige bestehende Methoden versuchen die Hörschwelle des Nutzers zu schätzen (z.B. Mimi Music App, Samsung Sound Adapt) und damit die Parameter für eine Klangpersonalisierung einzustellen (aus gleicher Hörschwelle folgt hierbei gleiche Klangeinstellung). Ein Problem hierbei ist, dass die Hörschwellenmessung auf nicht-kalibrierten Audiogeräten vorgenommen wird. Der verwendete Kopfhörer und damit der Übertragungsweg des Systems sind unbekannt. Deshalb kann keine absolute Hörschwellenmessung erfolgen, wie es mit einem kalibrierten Audiometer erfolgen würde, sondern eine individuelle Hörschwelle bezogen auf die akustischen Eigenschaften am Abspielgerät.

Die Einstellungen bei Hörgeräten erfolgt auf Grund der gemessenen Hörschwelle, jedoch wird hier die Anpassung durch einen Experten (Audiologen) vorgenommen. Der Hörgeräteträger kann zwar zwischen vorgegebenen Programmen wechseln, aber keine freie Änderung der Klangparameter vornehmen.

Bei Anpassmethoden, die nicht die Hörschwelle bestimmen, werden Voreinstellungen für die Klangparameter dem Nutzer auf einer Bedienoberfläche zur Verfügung gestellt, mit der der Nutzer sich den Klang des aktuellen Signals einstellen kann (z.B. Soundhawk App, EARs, Earmachine, BioAid). Diese Anpasskonzepte berücksichtigen dabei aber nicht die individuelle Hörschwelle des Nutzers, die bei leisen Passagen eines Musikstückes eine Rolle spielt.

Bei der Klanganpassung in Automobilen wurde ein System vorgestellt (vgl. Christoph, Markus. "Noise dependent equalization control." Audio Engineering Society Conference: 48th International Conference: Automotive Audio. Audio Engineering Society, 2012), das bei einem gegebenen Hintergrundrauschen eine frequenzabhängige Verstärkung auf das Zielsignal anwendet, damit der Klangeindruck bei veränderlichem Hintergrundrauschen erhalten bleibt. Diese Lösung berücksichtig aber keine individuellen Unterschiede des Zuhörers, die z.B. auf Grund des individuellen Hörvermögens vorhanden sind.

Ferner bildet die US 8,165,327 B2 Stand der Technik. Diese offenbart ein Verfahren zum Erzeugen eines akustischen Signals mit einem Hörgerät. Akustische Töne eines Hörgeräts müssen an die entsprechende Situation angepasst werden, um das Hören mit den Hörgeräten zu verbessern.

Es sind Verfahren für Automotive-Audiosysteme patentiert ("Sound reproduction device including auditory scenario simulation"; z. B. US 9,445,169 oder JP 5898305), die dem Nutzer simulierte Fahr-/Nebengeräusche vorspielen, während dieser die individuellen Klangeinstellungen vornimmt (Trainingsphase), und dann in der Betriebsphase das echte Fahr-/ Nebengeräusch analysieren und automatisch die Klangeinstellungen auswählen, für die die simulierten Nebengeräusche am ehesten zu den realen Geräuschen passen. Deshalb besteht der Bedarf nach einem verbesserten Ansatz.

Aufgabe der vorliegenden Erfindung ist es ein Konzept zu schaffen, das die Hörcharakteristika und Hörpräferenzen eines Nutzers vollumfänglich berücksichtigt.

Die Aufgabe wird durch die unabhängigen Ansprüche gelöst. Ausführungsbeispiele gemäß der Erfindung schaffen ein Verfahren zur Einstellung von Parametern zur individuellen Anpassung eines Audiosignals. Dieses Einstellverfahren untergliedert sich in zwei Phasen: in der ersten Phase wird eine Art Hörtest durchgeführt, mit dem Ziel einen an die individuelle Hörschwelle (je Frequenzbereich) angepassten Pegel zu erhalten. Diese erste Phase wird unter Zuhilfenahme eines ersten Audiosignals, wie z. B. eines Testsignals oder auch eines realen Signals durchgeführt. In der zweiten Phase erfolgt dann eine Anpassung des (z. B. eigentlichen) zweiten Audiosignals durch Variieren eines Klanganpassungskennfeldes unter Berücksichtigung des Pegels für die individuellen Hörschwellen je Frequenzbereich. Im Detail: Das Verfahren umfasst die Schritte Durchführen eines ersten Hörtests und Durchführen einer Anpassung des zweiten Audiosignals. Der erste Hörtest umfasst die Unterschritte:
- Abspielen einer Vielzahl von ersten Audiosignalen mit unterschiedlichen Pegeln und für unterschiedliche Frequenzbereiche, um eine Vielzahl von ersten akustischen Signalen unterschiedlicher Schalldruckpegel in den unterschiedlichen Frequenzbereichen an ein Individuum auszugeben;
- Erhalten eines Feedbacks je Frequenzbereich der unterschiedlichen Frequenzbereiche von dem Individuum, welche der Vielzahl der ersten akustischen Signale oberhalb einer individuellen Hörschwelle liegt; und
- Verwenden, je Frequenzbereich der unterschiedlichen Frequenzbereiche, des niedrigsten Pegels der unterschiedlichen Pegel der Vielzahl der ersten Audiosignale, für das ein Feedback vorliegt, das das zugehörige erste akustische Audiosignal als ein akustisches Audiosignal oberhalb der individuellen Hörschwelle kennzeichnet, als Pegel für die individuelle Hörschwelle je Frequenzbereich der unterschiedlichen Frequenzbereiche.

Ausgehend von den so ermittelten frequenzabhängigen Pegeln für die individuelle (frequenzabhängige) Hörschwelle wird dann eine Anpassung mit folgenden Unterschritten durchgeführt:
- Abspielen des zweiten Audiosignals entsprechend einem von dem Individuum gewählten Gesamtlautstärkepegel unter Berücksichtigung eines Klanganpassungskennfelds, um ein nachbearbeitetes zweites akustisches Signal an das Individuum auszugeben;
- Variieren des Klanganpassungskennfelds bis das Individuum durch Interaktion anzeigt, dass keine weitere Variation des Klanganpassungskennfelds gewünscht ist. Hierbei definiert das Klanganpassungskennfeld eine individuelle Anhebung und/oder individuelle Absenkung der Ausgangspegel je Eingangspegel für unterschiedliche Frequenzbereiche. Die Pegel für die individuellen Hörschwellen je Frequenzbereich der unterschiedlichen Frequenzbereiche werden bei dem Klanganpassungskennfeld als minimale Ausgangspegel verwendet. Insofern sind also in dem Klanganpassungskennfeld die Pegel für die individuellen Hörschwellen (je Frequenzbereich) mitgespeichert.

Entsprechend dem Ausführungsbeispiel erfolgt die Speicherung so, dass dieses Klanganpassungskennfeld für die Wiedergabe des zweiten Audiosignals, d. h. also beispielsweise des aktuell abzuspielenden Audiosignals verwendet werden kann. Entsprechend weiterem Ausführungsbeispiel kann der Schritt des Anpassens zu einem späteren Zeitpunkt wiederholt werden, wenn beispielsweise eine Variation des Audiosignals gewünscht ist. Dieser Schritt wird entsprechend Ausführungsbeispielen als Klanganpassung online bzw. Nachregelung des aktuellen Signals bezeichnet und berücksichtigt weiterhin die bei dem ersten Hörtest ermittelten Pegel für die individuellen Hörschwellen je Frequenzbereich.

Ausführungsbeispielen der vorliegenden Erfindung liegt die Erkenntnis zugrunde, dass eine individuelle Klanganpassung unter Berücksichtigung der effektiven Hörschwellen durch ein zweiphasiges Verfahren erreicht werden kann. In der ersten Phase wird ein (offline) Hörtest zur Abschätzung der effektiven Hörschwelle durchgeführt, während in der zweiten Phase ein (offline oder online) Verfahren zur Klanganpassung von Musiksignalen abläuft. Optionaler Weise kann dieser Ansatz um eine dritte Phase zur Nachregelung des aktuellen Signals erweitert werden, wobei diese dritte Phase im Wesentlichen die Schritte der zweiten Phase wiederholt, um "online" (d.h. während des Betriebs) die Klangcharakteristik zu modifizieren. Hierzu muss nicht erneut die erste Phase durchlaufen werden, da die effektive Hörschwelle personenbezogen ist (und eine aus Sicht der jeweiligen Person sich nicht veränderliche Hörcharakteristik darstellt) und im Regelfall nicht neu bestimmt werden muss.

Das Abschätzen der effektiven Hörschwelle in der ersten Phase dient dazu, die untere Grenze bei der Signalpräsentation festzulegen. Das bedeutet also, dass das Klanganpassungskennfeld so definiert ist, dass bei der späteren Anwendung desselben auf das abzuspielende Audiosignal leise Signalanteile im gesamten Frequenzbereich weiterhin hörbar, d.h. oberhalb der individuellen effektiven Hörschwelle bleiben. Die Klangpersonalisierung wird durch eine von dem Nutzer getroffene Auswahl von Kompressionseinstellungen durchgeführt, wobei der Nutzer entsprechend weiteren Ausführungsbeispielen während des Anhörens live zwischen unterschiedlichen Klangeinstellungen variieren kann (Phase 3). In dieser Phase 2 bzw. in der wiederholten Phase 2, also der Phase 3, werden vorzugsweise Klanganpassungen bei mittleren und hohen Signalpegeln vorgenommen.

Von einer anderen Seite betrachtet heißt das, dass das Klanganpassungskennfeld, welches aus der ersten Phase zumindest die Pegel für die individuellen Hörschwellen (minimalen Pegel) umfasst, um die entsprechenden (Ausgangs-)Pegelanpassungen für mittlere und hohe Signalpegel (Eingangspegel) erweitert wird. Hierbei versteht es sich, dass sowohl die individuellen Hörschwellen in unterschiedlichen Frequenzbereichen definiert sind, als auch die Pegelanhebungen/-absenkungen für die mittleren und hohen Signalpegel frequenzselektiv variiert werden. Der wichtigste Vorteil ist die Berücksichtigung der individuellen Klangpräferenz und der individuellen effektiven Hörschwelle innerhalb einer Klangeinstellung. Die effektive Hörschwelle stellt sicher, dass leise Signalanteile hörbar bleiben. Im mittleren bis hohen Lautstärkebereich spielt die effektive Hörschwelle nur eine untergeordnete Rolle. Deshalb werden hier die Einstellungen der Klangpräferenz werden verwendet. Dieses Verfahren kombiniert die Berücksichtigung der effektiven individuellen Hörschwelle und der individuellen Klanganpassung fürs Musikhören unter den realen Abhörbedingungen.

Entsprechend Ausführungsbeispielen wird der Hörtest und die Anpassung unter Verwendung der von dem Individuum ausgewählten Audiogeräte und/oder in der akustischen Umgebung des Individuums durchgeführt. Das hat den Vorteil, dass hierfür nicht ein besonders kalibriertes Gerät notwendig ist und so auch direkt gerätespezifische Charakteristika mitberücksichtigt werden. Weiter werden auch Charakteristika durch den Abhörraum bzw. die Position des Individuums im Abhörraum, hierbei insbesondere der Abstand und die damit verbundene Dämpfung des Lautstärkepegels mitberücksichtigt. Dieser Ansatz bietet also den Vorteil, dass das Konzept nicht an eine spezielle Hardware gebunden ist und die vollständige Gehörsituation mitberücksichtigt wird. Bei Veränderung der Hörsituation kann das Verfahren erneut durchgeführt werden. An dieser Stelle sei auch angemerkt, dass entsprechend weiteren Ausführungsbeispielen das erste Audiosignal zur Ermittlung der individuellen Hörschwelle einen Signalanteil umfassen kann, der eine Simulation von Fahr- und/oder Nebengeräuschen ermöglicht.

Entsprechend weiteren Ausführungsbeispielen wird insbesondere der Schritt des Anpassens des zweiten Audiosignals für unterschiedliche Gesamtlautstärkepegel (also für unterschiedliche vom Benutzer gewählte Ausgangslautstärkepegel) wiederholt. Das ermöglicht, dass das Klanganpassungskennfeld je Gesamtlautstärkepegel gespeichert wird. Nach der erfolgreichen Durchführung ist der Lautstärkeregler des Audiosystems auf die individuelle Abhörsituation kalibriert, so dass das Signal erst auf der niedrigsten Stufe des Reglers unter die Hörschwelle fällt und damit unhörbar wird. Die maximale Lautstärkeeinstellung nutzt den gesamten Dynamikbereich des technischen Systems aus.

Insbesondere in der dritten Phase bzw. beim Durchführen der zweiten Phase, wäre es wünschenswert, dass die Anpassung kontinuierlich erfolgt. Deshalb erfolgt das Variieren des Klanganpassungskennfeldes durch kontinuierliches Anpassen zumindest einer Dimension des Klanganpassungskennfeldes, um so die resultierenden Klangcharakteristikveränderung über die Zeit und/oder Schalldruckpegelunterschiede über die Zeit zu glätten. Dieses Anpassen erfolgt typischerweise durch Interaktion mit dem Benutzer, der einen Regler, wie z. B. einen zweidimensionalen Verschieberegler oder drei Einzelregler für Höhen, Mitten und Tiefen betätigt. Mit einem ähnlichen Regler erfolgt auch die Interaktion zwischen Benutzer und der ausführenden Vorrichtung, bei der Bestimmung der effektiven Hörschwelle. An dieser Stelle sei angemerkt, dass das Signal für die Bestimmung der effektiven Hörschwelle beispielsweise ein Testsignal sein kann, während das zweite Audiosignal entweder ein Testsignal, oder ein komplexes Audiosignal (mit oder ohne eingeschränkten Frequenzbereich), oder auch ein aktuelles Musikstück sein kann.

Entsprechend Ausführungsbeispielen erfolgt das Variieren des Klanganpassungskennfeldes für Eingangswerte oberhalb des Pegels für die individuelle Hörschwelle und für Pegel unterhalb eines Pegels für die Limitierung. Hierbei können entsprechend weiteren Ausführungsbeispielen Eingangspegel von mindestens 10 dB oberhalb des jeweiligen Pegels für die individuelle Hörschwelle und/oder Pegel von mindestens 3 dB unterhalb der jeweiligen Pegel für eine Limitierung variiert werden. Dieser Pegelbereich ist besonders interessant, da dieser die Klangcharakteristik bestimmt. Entsprechend weiteren Ausführungsbeispielen erfolgt die Variation des Klanganpassungskennfeldes so, dass eine Maskierungsschwelle nicht unterschritten wird, wobei die Maskierungsschwelle abhängig von der individuellen Hörschwelle ist.

Das Anpassen bzw. das Anwenden des Klanganpassungskennfeldes erfolgt unter Zuhilfenahme eines Multibandkompressors, der ausgebildet ist in Abhängigkeit des Klanganpassungskennfeldes das zweite Audiosignal im Hinblick auf die Ausgangspegel in den unterschiedlichen Frequenzbereichen nachzubearbeiten.

Ein weiteres Ausführungsbeispiel bezieht sich auf ein Computerprogramm zur Durchführung eines der oben erläuterten Verfahren.

Ein weiteres Ausführungsbeispiel gemäß der Erfindung bezieht sich auf eine Vorrichtung, das die Einstellung von Parametern zur individuellen Anpassung des Audiosignals durchführen kann. Diese Vorrichtung umfasst eine initiale Anpassungsstufe mit Mitteln zur Durchführung der Unterschritte des Hörtestes sowie eine weitere Anpassungsstufe zur Anpassung des zweiten Audiosignals mit Mitteln zur Durchführung der Unterschritte des Anpassens.

Weiterbildungen sind in den Unteransprüchen definiert. Ausführungsbeispiele der Erfindung werden nachfolgend anhand der beiliegenden Zeichnungen erläutert. Es zeigen:
- Fig. 1a: ein schematisches Flussdiagramm eines Verfahrens zur Einstellung von Parametern zur individuellen Anpassung gemäß einem Ausführungsbeispiel;
- Fig. 1b: eine schematische Darstellung einer Kompressionskennlinie in einem Band zur Illustration der Parameter für die individuelle Anpassung gemäß Ausführungsbeispielen;
- Fig. 1c: eine schematische Darstellung eines dreidimensionalen Klanganpassungskennfeldes gemäß einem Ausführungsbeispiel;
- Fig. 2: eine schematische Darstellung der Individualisierungsstufen bei einem Verfahren zur individuellen Anpassung gemäß Ausführungsbeispielen; und
- Fig. 3: eine schematische Darstellung einer Benutzerschnittstelle zur Anpassung eines Audiosignals gemäß weiteren Ausführungsbeispielen.

Bevor nachfolgend Ausführungsbeispiele der vorliegenden Erfindung anhand der Figuren erläutert werden, sei darauf hingewiesen, dass gleiche Elemente und Strukturen mit gleichen Bezugszeichen versehen sind, so dass die Beschreibung derer aufeinander anwendbar bzw. austauschbar ist.

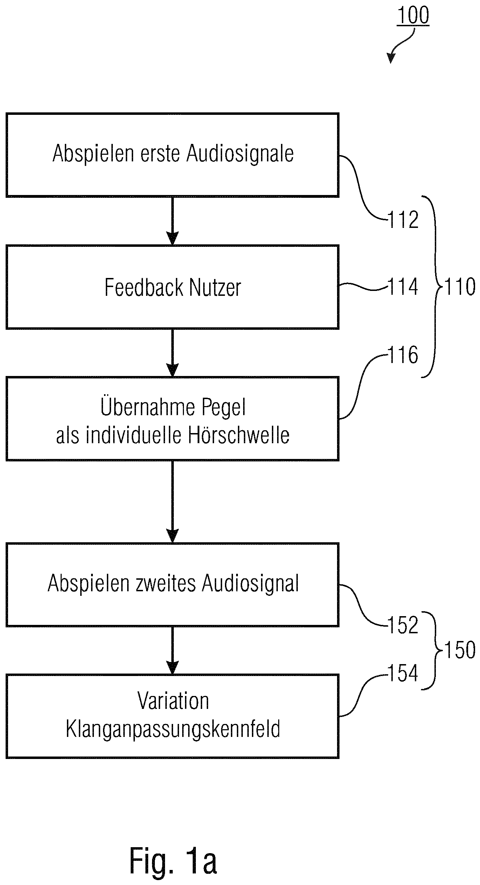

Fig. 1a zeigt ein Verfahren 100 mit den zwei Phasen 110 und 150. In der ersten Phase 110 wird ein erste Hörtest durchgeführt, der die Zielsetzung hat Pegel für individuelle Hörschwellen je Frequenzbereich (bezogen auf unterschiedliche Frequenzbereiche) zu ermitteln. Die Phase 150 dient zur Anpassung und hat die Zielsetzung ein Klanganpassungskennfeld zur Klangpersonalisierung unter Berücksichtigung der vorermittelten individuellen Hörschwellen zu erhalten.

In der Phase 110 werden im Wesentlichen folgende drei Schritte 112, 114 und 116 durchgeführt. Bei dem Schritt 112 erfolgt ein Abspielen eines ersten Audiosignals mit unterschiedlichen Pegeln, z. B. mit abnehmenden Pegeln. Dieses erste Audiosignal wird bevorzugter Weise für unterschiedliche Frequenzbereiche abgespielt, um in den unterschiedlichen Frequenzbereichen das erste akustische Signal mit unterschiedlichen Schaltpegeln an das Individuum auszugeben. Das Individuum hört dieses und gibt dann in dem Schritt 114 ein Feedback je Frequenzbereich. Das Feedback kennzeichnet das kleinste oder allgemein die ersten akustischen Signale oberhalb einer individuellen Hörschwelle. Beispielsweise kann der Benutzer/das Individuum solange das Erhalten des ersten akustischen Signals quittieren, bis er es nicht mehr hört. Der hierfür verwendete niedrigste Pegel je Frequenzbereich, für welchen ein Feedback vorliegt, dass das zugehörige akustische Signal oberhalb der individuellen Hörschwelle liegt, wird als Pegel für die individuelle Hörschwelle je Frequenzbereich übernommen, wie mit dem Schritt 116 symbolisiert ist, da die individuelle Hörschwelle frequenzselektiv ist, wird dieser erste Hörtest für unterschiedliche Frequenzbereiche, z. B. einen hohen und einen niedrigen Frequenzbereich oder für eine Vielzahl von unterschiedlichen Frequenzbereichen (20 bis 100 Hz, 100 bis 400 Hz, 400 bis 4.000 Hz, 4.000 bis 10.000 Hz, 10.000 bis 20.000 Hz) wiederholt. Beispielsweise kann hierfür jeweils ein speziell präpariertes Musiksignal (z. B. ein bandbegrenztes Musiksignal) als erstes Audiosignal verwendet werden, so dass eine effektive Hörschwelle für Musiksignale ermittelt wird, die von der audiometrischen Hörschwelle, die mit Sinustönen gemessen wird, abweichen kann.

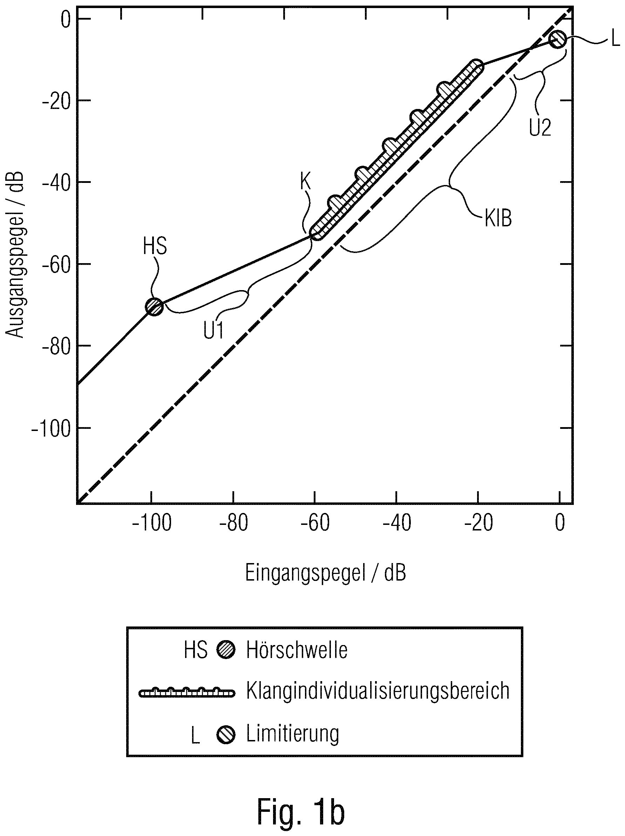

Das Ergebnis der Phase 110 wird nachfolgend anhand von Fig. 1b erläutert. Fig. 1b zeigt ein Diagramm des Ausgangspegels gegenüber den Eingangspegeln. Bei einer linearen Vorverstärkung wird ein Eingangspegel 1 zu 1 auf einen Ausgangspegel abgebildet. Wenn man beispielsweise von einem -100dB ausgeht, wird ohne Klanganpassung ein -100dB-Signal ausgegeben. Ein derartiges Signal liegt allerdings unter der Hörschwelle, die mit Bezugszeichen HS gekennzeichnet ist. Diese Hörschwelle HS, welche in diesem Ausführungsbeispiel rund -70dB hat, ist personenindividuell und frequenzindividuell. Insofern ist dieses Diagramm nur für ein Frequenzbereich und auch für eine Person gültig.

Ausgehend von dieser ermittelten Hörschwelle erfolgt nun eine Anhebung der Signale zugehörig zu den niedrigen Eingangspegeln, so dass die Ausgangspegel ausgehend von Eingangspegeln im Bereich zwischen -100 dB und -70 dB oder im Bereich zwischen -100 dB und -60 dB immer oberhalb der Hörschwelle HS liegen.

In der zweiten Phase 150 erfolgt nun eine Anpassung eines zweiten Audiosignals. Diese Klanganpassung betrifft bevorzugter Weise die mittleren und hohen Signalpegel über den gesamten Frequenzbereich, wobei auch hier die Anpassung über unterschiedliche Frequenzbereiche des gesamten Frequenzspektrums erfolgt. Hierbei können die Frequenzbereiche analog zu der Anpassung in der Phase 110 oder auch unabhängig hiervon in eins der Frequenzbänder untergliedert sein.

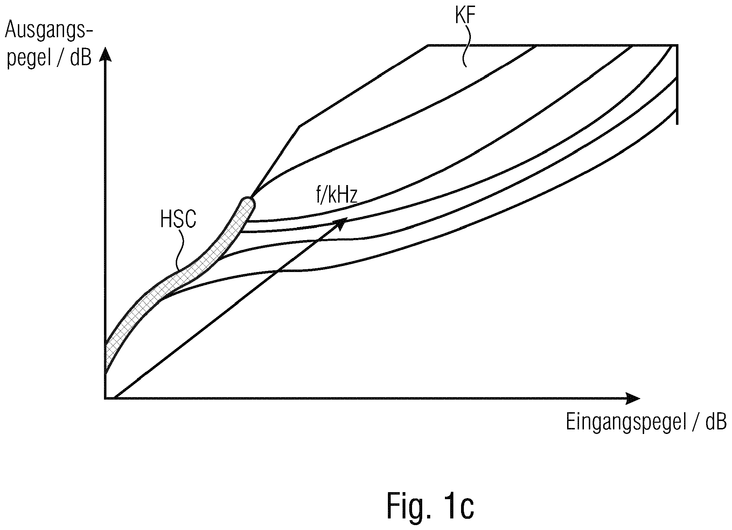

Die Phase 150 umfasst die zwei Schritte 152 und 154. Der Schritt 152 bezieht sich auf das Abspielen eines zweiten Audiosignals, wie z. B. eines aktuellen Musiksignals, in Entsprechung eines von dem Individuum gewählten Gesamtlautstärkepegels. Dies spielt deshalb eine Rolle, weil die Klangpräferenzen in unterschiedlichen Lautstärkebereichen für den Hörer häufig unterschiedlich sind, so dass insbesondere die mittels der Phase 150 ermittelten Anpassungen bevorzugt in einem Gesamtlautstärkepegelbereich Anwendung finden. Der Hintergrund hierzu ist, dass ein Nutzer bei einer niedrigen Gesamtlautstärke gegebenenfalls eine Badewannenkurve bevorzugt (angehobene Bässe und Höhen), während der gleiche Nutzer bei einer höheren Gesamtlautstärke gegebenenfalls zu einer linearen Verstärkung tendiert, so dass die Bässe und Höhen nicht in einen unangenehmen Ausgangspegelbereich angehoben werden. Des Weiteren sei angemerkt, dass dieses zweite Audiosignal unter Berücksichtigung eines Klanganpassungskennfeldes abgespielt wird, um ein in Abhängigkeit von diesem Klanganpassungskennfeld nachbearbeitetes zweites Audiosignal auszugeben. Fig. 1b zeigt einen Ausschnitt des Klanganpassungskennfeldes, nämlich die Kennlinie K bei einem speziellen Frequenzbereich, z.B. ein Bereich um 1000Hz (800-1500Hz). Das gesamte Klanganpassungskennfeld über alle relevanten /hörbaren Frequenzen (30Hz bis 20kHz oder 20Hz bis 22kHz) ist in Fig. 1c dargestellt.

Wie ausgehend von Fig. 1c ersichtlich ist, umfasst das Klanganpassungskennfeld drei Dimensionen, nämlich, wie auch Fig. 1b schon zeigte, ein Mapping von Eingangspegeln gegenüber Ausgangspegeln sowie in der dritten Dimension den Frequenzbereich in kHz. Auch wenn es nicht dargestellt ist, sei an dieser Stelle angemerkt, dass dieses Klanganpassungskennfeld natürlich auch um eine vierte Dimension, nämlich den Gesamtlautstärkepegel erweitert sein kann.

In dem Schritt 154 variiert nun der Benutzer des Klanganpassungskennfelds KF, z. B. durch Anhebung von Höhen, Mitten oder Tiefen. Durch diese Variation ändert sich die Nachverarbeitung des zweiten akustischen Signals, so dass der Benutzer ein direktes Feedback erhält. Was allerdings bei der Variation des Klanganpassungsfeldes berücksichtigt bleibt, ist, dass die individuellen Hörschwellen, welche beim dreidimensionalen Kennfeld mit dem Bezugszeichen HSL gekennzeichnet ist, nicht unterschritten wird und das Klanganpassungskennfeld also durch die Linie HSL in eine Region begrenzt ist. Nachfolgend wird Bezug nehmend auf Fig. 1b die Variation 154 erläutert. Diese in Fig. 1b dargestellte Kompressionskennlinie K für ein Band des Kompressors kann in dem so genannten Klangindividualisierungsbereich KIB zwischen den zwei schwarzen Punkten variiert werden. Dieser Klangindividualisierungsbereich liegt typischerweise zwischen den so genannten Limitierungspunkt L und der Hörschwelle HS, wobei entsprechend bevorzugtem Ausführungsbeispiel ein gewisser Abstand, wie z. B. 10 dB zu HS und 3 dB zu L vorherrscht. Wie zu erkennen ist, ist der Klangindividualisierungsbereich folglich im oberen Bereich angeordnet. Der untere Bereich wird durch die mittels des Hörtests ermittelte Hörschwelle HS bestimmt bzw. dominiert. Ausgehend von dieser Hörschwelle erfolgt dann ein Übergang zu dem unteren Punkt des Klangindividualisierungsbereiches in dem Bereich U1, der sich in Abhängigkeit von den Punkten HS und dem Klangindividualisierungsbereich KIB verschiebt. Wenn ein Benutzer in diesem Frequenzbereich beispielsweise eine Anhebung des Frequenzbereichs wünscht, wird der Klangindividualisierungsbereich KIB entweder partiell oder vollumfänglich nach oben verschoben, während bei einer gewünschten Absenkung der Pegel in diesem Frequenzbereich eine Verschiebung nach unten erfolgt. Wie bereits angedeutet, kann entsprechend Ausführungsbeispielen der Bereich KIB als lineares Element (keine Kompression) verschoben werden, oder auch eine Untergliederung des Bereichs KIB in unterschiedliche andere Bereiche erfolgen. Ausgehend von der Verschiebung dieses mittleren Bereichs im Rahmen der Klanganpassung erfolgt eine durch den Nutzer eingestellte Frequenzgewichtung. In Abhängigkeit von der Verschiebung von KIB erfolgt nun eine Anpassung des Bereiches U2, so dass hier ein Übergang bis zu der Limitierung L erfolgt.

Bezüglich des Diagramms sei noch angemerkt, dass der Abstand des Klangindividualisierungsbereichs KIB von der Diagonalen (gestrichelte Linie) darstellt, ob eine Erhöhung oder eine Reduzierung des Ausgangspegels gegenüber dem Eingangspegel erfolgt. Im hier dargestellten Ausführungsbeispiel liegt also immer eine Erhöhung vor, bis auf den Bereich zwischen einem Eingangspegel von -10 bis 0 dB (Limitierung).

Ausgehend von der so eingestellten Kompressionskennlinie erfolgt dann eine Nachverarbeitung des zweiten Audiosignals, beispielsweise wie folgt: ist der Präsentationspegel nahe der Hörschwelle HS, bestimmt die effektive Hörschwelle die Verarbeitung. Ist der Präsentationspegel deutlich oberhalb der Schwelle HS, bestimmt die Klangpersonalisierung im Bereich KIB die Verarbeitung. Mit Hilfe dieses Verfahrens wird erreicht, dass bei einer Verringerung der Abhörlautstärke alle Signalanteile im hörbaren Bereich bleiben und nicht einzelne Frequenzbereiche unterhalb der Hörschwelle fallen. Im mittleren bis hohen Lautstärkebereich dominiert die individuelle Klangpräferenz die Einstellung der Signalverarbeitung.

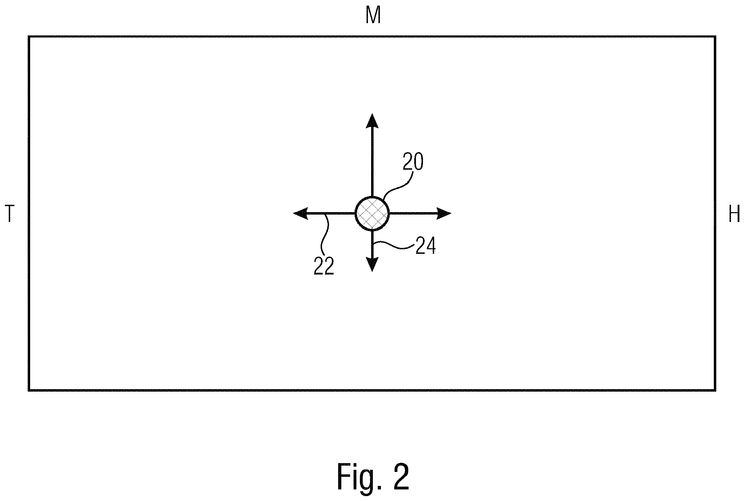

Diese Anpassung wir über mehrere Frequenzbereiche durchgeführt, so dass im Resultat das Kennfeld KF erhalten wird. Fig. 2 zeigt einen möglichen Regler für die Phase der Anpassung. Hierbei wird von einem zweidimensionalen Feld ausgegangen, das in einer ersten Dimension (vgl. Pfeil 22) es ermöglicht, dass Klanganpassung mehr Richtung Tiefen oder mehr Richtung Höhen erfolgt, je nachdem, ob der Einstellpunkt 20 in Richtung T (Tiefen) oder in Richtung H (Höhen) bewegt wird. Eine Bewegung des 20 in Richtung T führt zu einer Anhebung des Bereiches KIB für ein tieffrequentes Frequenzband (z. B. im Frequenzband zwischen 20 und 150 Hz. Eine Bewegung in Richtung H reduziert einerseits den Bereich KIB im tieffrequenten Frequenzband und erhöht andererseits die Anhebung durch KIB in einem hochfrequenten Frequenzband (z. B. der Bereich zwischen 1.000 und 20.000 Hz). Um nun den Mittenbereich zwischen 150 und 1.000 Hz anzuheben, wird der Punkt 20 entlang der Achse 24 in Richtung M (Mitten) verschoben, was zu einer Anhebung von KIB für das mittlere Frequenzband führt.

Um nun die Höhen und die Tiefen unabhängig voneinander regeln zu können, kann anstelle des hier dargestellten Reglers ein Regler vergleichbar mit einem Equalizer, d. h. also eine Anordnung von drei Reglern für T, M und H individuell voneinander vorgesehen sein.

Alle Regler ermöglichen die Variationen des Klanganpassungskennfeldes, wie in Bezug auf den Schritt 154 beschrieben.

Da unter Umständen mehrere Kennlinien zu mehreren Lautstärkepegeln (Gesamtlautstärkepegel) sinnvoll sind, kann entsprechen weiteren Ausführungsbeispielen ein Speichern der Klanganpassungskennfelder erfolgen, die dann unterschiedlichen Gesamtlautstärkepegeln zugeordnet sind. Auch kann eine Speicherung unterschiedlicher Klanganpassungskennlinien für unterschiedliche Signale (z. B. ein Sprachsignal und ein Musiksignal) oder für unterschiedliche Musikrichtungen (Klassik versus Pop) hinterlegt werden.

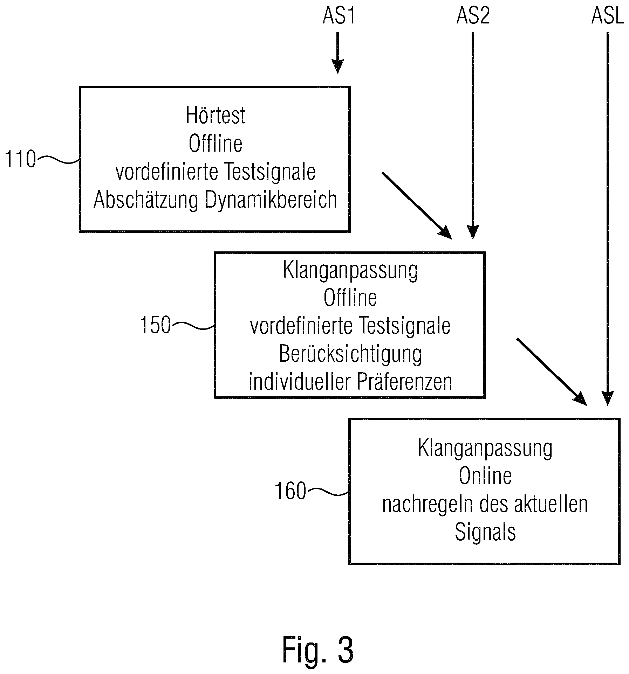

Entsprechend weiteren Ausführungsbeispielen kann diese Phase 150 wiederholt werden, wie nachfolgend Bezug nehmend auf Fig. 3 dargestellt wird. Fig. 3 illustriert die zwei Phasen 110, 150 sowie eine zugehörige weitere Phase 160, in welcher eine Nachregulierung des aktuellen Signals (aktuelles Musiksignal ASL als zweites Audiosigna)l erfolgt. Die Phase 160 ist im Wesentlichen vergleichbar mit der Phase 150, wobei der Unterschied darin liegt, dass hier ein aktuelles Musiksignal ASL verwendet wird und nicht, wie in der Phase 150 die Klanganpassung offline unter Zuhilfenahme von vordefinierten Testsignalen (AS2) erfolgt.. Bei dieser dritten Phase 160 kann auch von einer so genannten Live-Anpassung gesprochen werden. Bezugnehmend auf die Phase 110 sei angemerkt, dass hier ebenfalls ein Testsignals (vgl. Bezugszeichen AS1) verwendet wird

Entsprechend weiteren Ausführungsbeispielen erfolgt sowohl die Phase 110, als auch die Phase 150 und (selbstverständlicher Weise) die Phase 160 mit dem tatsächlichen akustischen Pfad, so dass der Einfluss von Hintergrundgeräuschen und das individuelle Hörvermögen des Benutzers bei der Ermittlung des Klanganpassungskennfeldes mitberücksichtigt wird.

Im Zusammenhang mit den Ausführungsbeispielen aus Fig. 1b und 1c sei angemerkt, dass sich die entsprechend untergliederten Frequenzbereiche auch gegenseitig beeinflussen können. Hierbei erfolgt beispielsweise eine Maskierung des einen Frequenzbereiches, wenn der andere Frequenzbereich extrem erhöht gegenüber dem anderen Frequenzbereich ist. Deshalb kann entsprechend Ausführungsbeispielen durch die Abschätzung von Maskierschwellen bestimmt werden, welche Signalteile zwar oberhalb der Hörschwelle HS liegen, aber aufgrund von Maskierung von benachbarten Bändern nicht hörbar sind. Bei diesen Frequenzanteilen kann dann die hörschwellenbasierte reduziert werden oder umgekehrt die maskierenden Bänder angehoben werden, damit diese Anteile auch nach der Verarbeitung weiterhin maskiert und damit unhörbar bleiben.

Ein weiteres Ausführungsbeispiel bezieht sich auf eine Vorrichtung, die das Verfahren 100 anwendet. Diese Vorrichtung umfasst neben Testsignalquellen und Mitteln zur Feedbackeingabe auch Mittel zu Klanganpassung. Bei diesen Mitteln zur Klanganpassung kann es sich beispielsweise um einen so genannten Multiband-Dynamik-Kompressor handeln, der ausgebildet ist, eine Klanganpassung entsprechend des Klanganpassungskennfeldes durchzuführen.

Bei oben genannten Ausführungsbeispielen wurde davon ausgegangen, dass der gesamte Frequenzbereich in drei Teilbereiche (Tief-, Mittel- und Hochton) eingeteilt wird. Selbstverständlich ist so eine Einteilung in wesentlich mehr Teilbereiche, z. B. in Oktavschritten oder vergleichbar möglich.

Bezugnehmend auf das Ausführungsbeispiel aus Fig. 3c sei angemerkt, dass bevorzugter Weise für die Phasen 110 und 150 der akustische Pfad unter Realbedingungen genutzt wird. Wenn man beispielsweise von einem Autoradio ausgeht, sind die Realbedingungen dann erfüllt, wenn beispielsweise Nebengeräusche, z. B. Fahrgeräusche vorliegen. Um eine Anpassung im Stand dennoch durchzuführen, kann entsprechend weiteren Ausführungsbeispielen eine Simulation von Hörszenen des Hörtests 110 oder während der Klanganpassung 150 erfolgen. Hierbei wird dann zusätzlich zu den eigentlich vordefinierten Hörsignalen auch noch ein Simulationssignal, wie z. B. ein Umgebungsgeräusch oder Fahrgeräusch mit dem Testsignal untergemischt, so dass diese Nebengeräusche bei der Messung bzw. der Abschätzung der effektiven Hörschwelle und der Anpassung mit einbezogen werden kann. Hierbei liegt der Vorteil darin, dass die Klangeinstellung im Wiedergabebetrieb des Audiosystems unter realitätsnahen Umgebungsbedingungen erfolgt und so sichergestellt ist, dass weder die individuelle Hörschwelle, noch ein vorliegendes Nebengeräusch die Hörbarkeit bei leisen Abhörlautstärken reduziert.

Alle oben erläuterten Ausführungsbeispiele haben gemein, dass sowohl eine Interaktion mit dem Benutzer vorliegt, um die effektive Hörschwelle des Nutzers zu bestimmen, als auch eine Interaktion des Benutzers um das Audiosignal oberhalb der Hörschwelle anzupassen. Beide diese subjektiven Messergebnisse unter Zuhilfenahme des Individuums werden dann bei der Signalverarbeitung berücksichtigt, so dass bei der Klangeinstellung bei leisen Abhörlautstärken ein individueller Einfluss, insbesondere von der individuellen Hörschwelle vorliegt, während andere Einstellungen (z. B. EQ) bei mittleren und hohen Abhörlautstärken von der Hörschwelle abhängen. Um Bezug zu nehmen auf Fig. 1b, gibt es zwischen der Hörschwelle HS und dem Klangindividualisierungsbereich KIB ein Übergangsbereich U1, der sowohl von der Hörschwelle als auch von dem KIB beeinflusst wird.

Das oben erläuterte Verfahren kann auf allen Geräten implementiert werden, die zur Wiedergabe von Audiosignalen, wie z. B. zum Musikhören geeignet sind. Insbesondere ist das Musikhören im Auto ein wesentliches Anwendungsgebiet, die es hier ein zeitlich variables Hintergrundrauschen vorliegt und es große individuelle Klangpräferenzen beim Musikhören gibt. Auch die Nutzung von Radio und Fernsehen zu Hause, das Musikhören im Flugzeug und auf mp3 Playern und Smartphones stellen weitere Anwendungsgebiete dar.

Obwohl manche Aspekte im Zusammenhang mit einer Vorrichtung beschrieben wurden, versteht es sich, dass diese Aspekte auch eine Beschreibung des entsprechenden Verfahrens darstellen, sodass ein Block oder ein Bauelement einer Vorrichtung auch als ein entsprechender Verfahrensschritt oder als ein Merkmal eines Verfahrensschrittes zu verstehen ist. Analog dazu stellen Aspekte, die im Zusammenhang mit einem oder als ein Verfahrensschritt beschrieben wurden, auch eine Beschreibung eines entsprechenden Blocks oder Details oder Merkmals einer entsprechenden Vorrichtung dar. Einige oder alle der Verfahrensschritte können durch einen Hardware-Apparat (oder unter Verwendung eines Hardware-Apparats), wie zum Beispiel einen Mikroprozessor, einen programmierbaren Computer oder eine elektronische Schaltung ausgeführt werden. Bei einigen Ausführungsbeispielen können einige oder mehrere der wichtigsten Verfahrensschritte durch einen solchen Apparat ausgeführt werden.

Je nach bestimmten Implementierungsanforderungen können Ausführungsbeispiele der Erfindung in Hardware oder in Software implementiert sein. Die Implementierung kann unter Verwendung eines digitalen Speichermediums, beispielsweise einer Floppy-Disk, einer DVD, einer Blu-ray Disc, einer CD, eines ROM, eines PROM, eines EPROM, eines EEPROM oder eines FLASH-Speichers, einer Festplatte oder eines anderen magnetischen oder optischen Speichers durchgeführt werden, auf dem elektronisch lesbare Steuersignale gespeichert sind, die mit einem programmierbaren Computersystem derart zusammenwirken können oder zusammenwirken, dass das jeweilige Verfahren durchgeführt wird. Deshalb kann das digitale Speichermedium computerlesbar sein.

Manche Ausführungsbeispiele gemäß der Erfindung umfassen also einen Datenträger, der elektronisch lesbare Steuersignale aufweist, die in der Lage sind, mit einem programmierbaren Computersystem derart zusammenzuwirken, dass eines der hierin beschriebenen Verfahren durchgeführt wird.

Allgemein können Ausführungsbeispiele der vorliegenden Erfindung als Computerprogrammprodukt mit einem Programmcode implementiert sein, wobei der Programmcode dahingehend wirksam ist, eines der Verfahren durchzuführen, wenn das Computerprogrammprodukt auf einem Computer abläuft.

Der Programmcode kann beispielsweise auch auf einem maschinenlesbaren Träger gespeichert sein.

Andere Ausführungsbeispiele umfassen das Computerprogramm zum Durchführen eines der hierin beschriebenen Verfahren, wobei das Computerprogramm auf einem maschinenlesbaren Träger gespeichert ist.

Mit anderen Worten ist ein Ausführungsbeispiel des erfindungsgemäßen Verfahrens somit ein Computerprogramm, das einen Programmcode zum Durchführen eines der hierin beschriebenen Verfahren aufweist, wenn das Computerprogramm auf einem Computer abläuft.

Ein weiteres Ausführungsbeispiel der erfindungsgemäßen Verfahren ist somit ein Datenträger (oder ein digitales Speichermedium oder ein computerlesbares Medium), auf dem das Computerprogramm zum Durchführen eines der hierin beschriebenen Verfahren aufgezeichnet ist.

Ein weiteres Ausführungsbeispiel des erfindungsgemäßen Verfahrens ist somit ein Datenstrom oder eine Sequenz von Signalen, der bzw. die das Computerprogramm zum Durchführen eines der hierin beschriebenen Verfahren darstellt bzw. darstellen. Der Datenstrom oder die Sequenz von Signalen kann bzw. können beispielsweise dahingehend konfiguriert sein, über eine Datenkommunikationsverbindung, beispielsweise über das Internet, transferiert zu werden.

Ein weiteres Ausführungsbeispiel umfasst eine Verarbeitungseinrichtung, beispielsweise einen Computer oder ein programmierbares Logikbauelement, die dahingehend konfiguriert oder angepasst ist, eines der hierin beschriebenen Verfahren durchzuführen.

Ein weiteres Ausführungsbeispiel umfasst einen Computer, auf dem das Computerprogramm zum Durchführen eines der hierin beschriebenen Verfahren installiert ist.

Ein weiteres Ausführungsbeispiel gemäß der Erfindung umfasst eine Vorrichtung oder ein System, die bzw. das ausgelegt ist, um ein Computerprogramm zur Durchführung zumindest eines der hierin beschriebenen Verfahren zu einem Empfänger zu übertragen. Die Übertragung kann beispielsweise elektronisch oder optisch erfolgen. Der Empfänger kann beispielsweise ein Computer, ein Mobilgerät, ein Speichergerät oder eine ähnliche Vorrichtung sein. Die Vorrichtung oder das System kann beispielsweise einen Datei-Server zur Übertragung des Computerprogramms zu dem Empfänger umfassen.

Bei manchen Ausführungsbeispielen kann ein programmierbares Logikbauelement (beispielsweise ein feldprogrammierbares Gatterarray, ein FPGA) dazu verwendet werden, manche oder alle Funktionalitäten der hierin beschriebenen Verfahren durchzuführen. Bei manchen Ausführungsbeispielen kann ein feldprogrammierbares Gatterarray mit einem Mikroprozessor zusammenwirken, um eines der hierin beschriebenen Verfahren durchzuführen. Allgemein werden die Verfahren bei einigen Ausführungsbeispielen seitens einer beliebigen Hardwarevorrichtung durchgeführt. Diese kann eine universell einsetzbare Hardware wie ein Computerprozessor (CPU) sein oder für das Verfahren spezifische Hardware, wie beispielsweise ein ASIC.

Die oben beschriebenen Ausführungsbeispiele stellen lediglich eine Veranschaulichung der Prinzipien der vorliegenden Erfindung dar. Es versteht sich, dass Modifikationen und Variationen der hierin beschriebenen Anordnungen und Einzelheiten anderen Fachleuten einleuchten werden. Deshalb ist beabsichtigt, dass die Erfindung lediglich durch den Schutzumfang der nachstehenden Patentansprüche und nicht durch die spezifischen Einzelheiten, die anhand der Beschreibung und der Erläuterung der Ausführungsbeispiele hierin präsentiert wurden, beschränkt sei.

## Patentansprüche

1. Verfahren zur Einstellung von Parametern zur individuellen Anpassung eines Audiosignals, mit folgenden Schritten:
Durchführen eines ersten Hörtests (110) mit den Unterschritten:
Abspielen (112) einer Vielzahl von ersten Audiosignalen (AS1) mit unterschiedlichen Pegeln und für unterschiedliche Frequenzbereiche, um eine Vielzahl von ersten akustischen Signalen unterschiedlicher Schalldruckpegel in den unterschiedlichen Frequenzbereichen an ein Individuum auszugeben;
Erhalten eines Feedbacks (114) je Frequenzbereich der unterschiedlichen Frequenzbereiche von dem Individuum, welche der Vielzahl der ersten akustischen Signale oberhalb einer individuellen Schwelle (HS) liegt; und
Verwenden (116), je Frequenzbereich der unterschiedlichen Frequenzbereiche, des niedrigsten Pegels der unterschiedlichen Pegel der Vielzahl der ersten Audiosignale, für das ein Feedback vorliegt, das das zugehörige erste akustische Audiosignal als ein akustisches Audiosignal oberhalb der individuellen Schwelle (HS) kennzeichnet, als Pegel für die individuelle Schwelle (HS) je Frequenzbereich der unterschiedlichen Frequenzbereiche;
Durchführen einer Anpassung(150) eines zweiten Audiosignals (AS2) mit den Unterschritten:
Abspielen (152) des zweiten Audiosignals (AS2) entsprechend einem von dem Individuum gewählten Gesamtlautstärkepegel unter Berücksichtigung eines Klanganpassungskennfelds (KF), um ein nachbearbeitetes zweites akustisches Signal an das Individuum auszugeben; und
Variieren (154) des Klanganpassungskennfelds (KF) mittels einer Benutzerschnittstelle oder eines Reglers bis das Individuum durch Interaktion anzeigt, dass keine weitere Variation des Klanganpassungskennfelds (KF) gewünscht ist;
wobei das Klanganpassungskennfeld (KF) eine individuelle Anhebung und/oder individuelle Absenkung der Ausgangspegel je Eingangspegel für unterschiedliche Frequenzbereiche definiert; wobei im Klanganpassungskennfeld (KF) die Pegel für die individuellen Schwellen (HS) je Frequenzbereich der unterschiedlichen Frequenzbereiche als minimale Ausgangspegel verwendet werden.

2. Verfahren gemäß Anspruch 1, wobei das Durchführen der Anpassung des zweiten Audiosignals (AS2) für unterschiedliche Gesamtlautstärkepegel wiederholt wird; und/oder wobei das Verfahren den Schritt des Speicherns des Klanganpassungskennfelds (KF) je Gesamtlautstärkepegel umfasst.

3. Verfahren gemäß Anspruch 1 oder 2, wobei der erste Hörtest und/oder die Anpassung unter Verwendung der von dem Individuum ausgewählten Audiogeräte und/oder in der akustischen Umgebung des Individuums erfolgt.

4. Verfahren gemäß einem der vorhergehenden Ansprüche, wobei das Variieren des Klanganpassungskennfelds (KF) durch kontinuierliches Anpassen zumindest einer Dimension des Klanganpassungskennfelds (KF) erfolgt, um so die resultierenden Klangcharakteristikveränderung über die Zeit und/oder Schalldruckpegelunterschiede über die Zeit zu glätten.

5. Verfahren gemäß einem der vorhergehenden Ansprüche, wobei das erste Audiosignal (AS1) und/oder das zweite Audiosignal (AS2( ein Testsignal, ein komplexes Audiosignal, ein komplexes Audiosignal mit einem eingeschränkten Frequenzbereich und/oder ein Musikstück ist.

6. Verfahren gemäß einem der vorhergehenden Ansprüche, wobei das Variieren des Klanganpassungskennfelds (KF) für Eingangspegel oberhalb des Pegels für die individuelle Schwelle (HS) und/oder für Pegel unterhalb eines Pegels für eine Limitierung erfolgt.

7. Verfahren gemäß Anspruch 6, wobei das Variieren des Klanganpassungskennfelds (KF) für Eingangspegel mindestens 10 dB oberhalb des jeweiligen Pegels für die individuelle Schwelle (HS) liegen und/oder mindestens 3 dB unterhalb der jeweiligen Pegel für eine Limitierung liegen.

8. Verfahren gemäß einem der vorhergehenden Ansprüche, wobei das Abspielen des zweiten Audiosignals (AS2) unter Zuhilfenahme eines Multibandkompressors erfolgt, wobei der Multibandkompressor in Abhängigkeit des Klanganpassungskennfelds (KF) das zweite Audiosignal (AS2) in Hinblick auf die Ausgangspegel in den unterschiedlichen Frequenzbereichen nachbearbeitet wird.

9. Verfahren gemäß einem der vorhergehenden Ansprüche, wobei das Klanganpassungskennfeld (KF) so gewählt sind, dass eine Maskierungsschwelle nicht unterschritten wird, wobei die Maskierungsschwelle abhängig von der individuellen Schwelle (HS) ist.

10. Verfahren gemäß einem der vorhergehenden Ansprüche, wobei beim Abspielen der Vielzahl der ersten Audiosignale (AS1) den Audiosignalen ein Signalanteil hinzugefügt wird, der eine Simulation von Fahr- und/oder Nebengeräuschen ermöglicht;

11. Verfahren gemäß einem der vorhergehenden Ansprüche, wobei das zweite Audiosignal (AS2, ASL) ein wiederzugebendes Audiosignal ist und die Anpassung des zweiten Audiosignals (AS2, ASL) im Betrieb wiederholt wird.

12. Computerprogramm mit einem Programmcode zur Ausführung eines Verfahrens der Ansprüche 1 bis 11, wenn das Programm auf einem Computer abläuft.

13. Vorrichtung zur Einstellung von Parametern zur individuellen Anpassung eines Audiosignals, mit folgenden Merkmalen:
einer initialen Anpassungsstufe zur Definition von Pegeln für eine individuelle Schwelle (HS) je Frequenzbereich der unterschiedlichen Frequenzbereiche mit Mitteln zum:
Abspielen einer Vielzahl von ersten Audiosignalen (AS1) mit unterschiedlichen Pegeln und für unterschiedliche Frequenzbereiche, um eine Vielzahl von ersten akustischen Signalen unterschiedlicher Schalldruckpegel in den unterschiedlichen Frequenzbereichen an ein Individuum auszugeben;
Erhalten eines Feedbacks je Frequenzbereich der unterschiedlichen Frequenzbereiche von dem Individuum, welche der Vielzahl der ersten akustischen Signale oberhalb der individuellen Schwelle (HS) liegt;
wobei, je Frequenzbereich der unterschiedlichen Frequenzbereiche, der niedrigste Pegel der unterschiedlichen Pegel der Vielzahl der ersten Audiosignale, für das ein Feedback vorliegt, das das zugehörige erste akustische Audiosignal als ein akustisches Audiosignal oberhalb der individuellen Schwelle (HS) kennzeichnet, als die Pegel für die individuellen Schwellen (HS) je Frequenzbereiche der unterschiedlichen Frequenzbereiche verwendet wird;
einer weiteren Anpassungsstufe zur Anpassung eines zweiten Audiosignals (AS2) mit Mittel zum:
Abspielen des zweiten Audiosignals (AS2) entsprechend eines von dem Individuum gewählten Gesamtlautstärkepegels unter Berücksichtigung eines Klanganpassungskennfelds (KF), um ein nachbearbeitetes zweites akustisches Signal an das Individuum auszugeben; und
Variieren des Klanganpassungskennfelds (KF) mittels einer Benutzerschnittstelle oder eines Reglers bis das Individuum durch Interaktion anzeigt, das keine weitere Variation des Klanganpassungskennfelds (KF) gewünscht ist;
wobei das Klanganpassungskennfeld (KF) eine individuelle Anhebung und/oder individuelle Absenkung der Ausgangspegel je Eingangspegel für unterschiedliche Frequenzbereiche definiert; wobei im Klanganpassungskennfeld (KF) die Pegel für die individuellen Schwellen (HS) je Frequenzbereich der unterschiedlichen Frequenzbereiche als minimale Ausgangspegel verwendet werden.

14. Vorrichtung nach Anspruch 13, wobei die Vorrichtung eine Benutzerschnittstelle zum Erhalten des Feedbacks und/ oder zur Variation des Klanganpassungskennfelds (KF) umfasst; oder
wobei die Vorrichtung eine Benutzerschnittstelle zur Erhalten des Feedbacks und/ oder zur Variation des Klanganpassungskennfelds (KF) umfasst und wobei die Benutzerschnittstelle ein oder mehrere Regler für die individuelle Anhebung und/oder individuelle Absenkung der Ausgangspegel in zumindest zwei Frequenzbereichen oder ein zweidimensionales Klanganpassungsfeld umfasst.

15. Vorrichtung gemäß einem der Ansprüche 13 bis 14, wobei die Vorrichtung Speicher zum Speichern des Klanganpassungskennfelds (KF) umfasst.

## Claims

1. Method for setting parameters for individual adaptation of an audio signal, comprising:
performing a first listening test (110) with the following substeps:
playing (112) a plurality of first audio signals (AS1) with different levels and for different frequency ranges to output a plurality of first acoustic signals of different sound pressure levels in the different frequency ranges to an individual;
obtaining feedback (114) per frequency range of the different frequency ranges from the individual, which of the plurality of first acoustic signals is above an individual listening threshold (HS); and
using (116), per frequency range of the different frequency ranges, the lowest level of the different levels of the plurality of first audio signals for which feedback is available, which characterizes the associated first acoustic audio signal as an acoustic audio signal above the individual listening threshold (HS), as a level for the individual listening threshold (HS) per frequency range of the different frequency ranges;
performing adaptation (150) of a second audio signal (AS2) with the following substeps:
playing (152) the second audio signal (AS2) according to a total volume level selected by the individual considering a sound adaptation characteristic map (KF) to output a post-processed second acoustic signal to the individual; and
varying (154) the sound adaptation characteristic map (KF) by means of a user interface or a control until the individual indicates by interaction that no further variation of the sound adaptation characteristic map (KF) is desired;
wherein the sound adaptation characteristic map (KF) defines an individual boost and/or individual cut of the output level per input level for different frequency ranges, wherein, the levels for the individual listening thresholds (HS) per frequency range of the different frequency ranges are used as minimum output levels in the sound adaptation characteristic map (KF).

2. Method according to claim 1, wherein performing the adaptation of the second audio signal (AS2) is repeated for different total volume levels; and/or wherein the method includes the step of storing the sound adaptation characteristic map (KF) per total volume level.

3. Method according to claim 1 or 2, wherein the first listening test and/or the adaptation is performed by using the audio devices selected by the individual and/or in the acoustic environment of the individual.

4. Method according to one of the preceding claims, wherein varying the sound adaptation characteristic map (KF) is performed by continuously adapting at least one dimension of the sound adaption characteristic map (KF) in order to smooth the resulting sound characteristic change over time and/or the sound pressure level differences over time.

5. Method according to one of the preceding claims, wherein the first audio signal (AS1) and/or the second audio signal (AS2) is a test signal, a complex audio signal, a complex audio signal with a limited frequency range and/or a piece of music.

6. Method according to one of the preceding claims, wherein varying the sound adaptation characteristic map (KF) is performed for input levels above the level for the individual threshold (HS) and/or for levels below a level for limitation.

7. Method according to claim 6, wherein varying the sound adaptation characteristic map (KF) for input levels is at least 10 dB above the respective level for the individual threshold (HS) and/or at least 3 dB below the respective level for limitation.

8. Method according to one of the preceding claims, wherein playing the second audio signal (AS2) is performed with the help of a multiband compressor, wherein the multiband compressor post-processes the second audio signal (AS2) with respect to the output levels in the different frequency ranges in dependence on the sound adaptation characteristic map (KF).

9. Method according to one of the preceding claims, wherein the sound adaptation characteristic map (KF) is selected such that the same does not fall below a masking threshold, wherein the masking threshold depends on the individual listening threshold (HS).

10. Method according to one of the preceding claims, wherein, when playing the plurality of first audio signals (AS1), a signal portion is added to the audio signals, which allows simulation of driving and/or ambient noise.

11. Method according to one of the preceding claims, wherein the second audio signal (AS2, ASL) is an audio signal to be replayed and adaptation of the second audio signal (AS2, ASL) is repeated during operation.

12. Computer program having a program code for performing a method of any one of claims 1 to 11 when the program runs on a computer.

13. Apparatus for setting parameters for individual adaptation of an audio signal, comprising:
an initial adaptation stage for defining levels for an individual listening threshold (HS) per frequency range of the different frequency ranges, comprising means for:
playing a plurality of first audio signals (AS1) having different levels and for different frequency ranges to output a plurality of first acoustic signals of different sound pressure levels in the different frequency ranges to an individual;
obtaining feedback per frequency range of the different frequency ranges from the individual, which of the plurality of first acoustic signals is above the individual listening threshold (HS); and
wherein, per frequency range of the different frequency ranges, the lowest level of the different levels of the plurality of first audio signals for which feedback is available, which characterizes the associated first acoustic audio signal as an acoustic audio signal above the individual listening threshold (HS), is used as the level for the individual listening thresholds (HS) per frequency range of the different frequency ranges;
a further adaptation stage for adapting a second audio signal (AS2), comprising means for:
playing the second audio signal (AS2) according to a total volume level selected by the individual considering a sound adaptation characteristic map (KF) to output a post-processed second acoustic signal to the individual; and
varying the sound adaptation characteristic map (KF) by means of a user interface or a control until the individual indicates by interaction that no further variation of the sound adaptation characteristic map (KF) is desired;
wherein the sound adaptation characteristic map (KF) defines an individual boost and/or individual cut of the output levels per input level for different frequency ranges; wherein the levels for the individual listening thresholds (HS) per frequency range of the different frequency ranges are used as minimum output levels in the sound adaptation characteristic map (KF).

14. Apparatus according to claim 13, wherein the apparatus includes a user interface for receiving the feedback and/or for varying the sound adaptation characteristic map (KF); or
wherein the apparatus includes a user interface for receiving the feedback and/or for varying the sound adaptation characteristic map (KF) and wherein the user interface includes one or several controls for individual boost and/or for individual cut of the output levels in at least two frequency ranges or a two-dimensional sound adaptation map.

15. Apparatus according to one of claims 13 or 14, wherein the apparatus includes a memory for storing the sound adaptation characteristic map (KF).

## Revendications

1. Procédé de réglage de paramètres pour l'adaptation individuelle d'un signal audio, aux étapes suivantes consistant à:
réaliser un premier test d'écoute (110), aux sous-étapes consistant à:
reproduire (112) une pluralité de premiers signaux audio (AS1) à différents niveaux et pour différentes plages de fréquences, pour sortir vers un individu une pluralité de premiers signaux acoustiques à différents niveaux de pression acoustique dans les différentes plages de fréquences;
recevoir de l'individu une rétroaction (114) par plage de fréquences des différentes plages de fréquences, pour savoir lequel parmi la pluralité de premiers signaux acoustiques se situe au-dessus d'un seuil individuel (HS); et
utiliser (116), par plage de fréquences des différentes plages de fréquences, le niveau le plus bas des différents niveaux de la pluralité de premiers signaux audio pour lequel existe une rétroaction qui caractérise le premier signal audio acoustique associé comme un signal audio acoustique au-dessus du seuil individuel (HS) comme niveaux pour le seuil individuel (HS) par plage de fréquences des différentes plages de fréquences;
réaliser une adaptation (150) d'un deuxième signal audio (AS2) aux sous-étapes consistant à:
reproduire (152) le deuxième signal audio (AS2) selon un niveau de volume sonore global sélectionné par l'individu, en tenant compte d'un diagramme caractéristique d'adaptation du son (KF), pour sortir vers l'individu un deuxième signal acoustique post-traité; et
faire varier (154) le diagramme caractéristique d'adaptation du son (KF) au moyen d'une interface d'utilisateur ou d'un régulateur jusqu'à ce que l'individu indique par interaction qu'il n'est pas souhaité d'autre variation du diagramme caractéristique d'adaptation du son (KF);
dans lequel le diagramme caractéristique d'adaptation du son (KF) définit une élévation individuelle et/ou un abaissement individuel des niveaux de sortie par niveau d'entrée pour différentes plages de fréquences; dans lequel, dans le diagramme caractéristique d'adaptation du son (KF), les niveaux pour les seuils individuels (HS) par plage de fréquences des différentes plages de fréquences sont utilisés comme niveaux de sortie minimaux.

2. Procédé selon la revendication 1, dans lequel la réalisation de l'adaptation du deuxième signal audio (AS2) est répétée pour différents niveaux de volume sonore globaux; et/ou dans lequel le procédé comporte l'étape consistant à mémoriser le diagramme caractéristique d'adaptation du son (KF) par niveau de volume sonore global.

3. Procédé selon la revendication 1 ou 2, dans lequel le premier test d'écoute et/ou l'adaptation est réalisé à l'aide des appareils audio sélectionnés par l'individu et/ou dans l'environnement acoustique de l'individu.

4. Procédé selon l'une des revendications précédentes, dans lequel la variation du diagramme caractéristique d'adaptation du son (KF) a lieu par adaptation en continu d'au moins une dimension du diagramme caractéristique d'adaptation du son (KF) pour lisser ainsi le changement de caractéristique du son résultant dans le temps et/ou les différences de niveau de pression acoustique dans le temps.

5. Procédé selon l'une quelconque des revendications précédentes, dans lequel le premier signal audio (AS1) et/ou le deuxième signal audio (AS2) est un signal de test, un signal audio complexe, un signal audio complexe à plage de fréquence restreinte et/ou une pièce de musique.

6. Procédé selon l'une des revendications précédentes, dans lequel la variation du diagramme caractéristique d'adaptation du son (KF) a lieu pour les niveaux d'entrée au-dessus du niveau pour le seuil individuel (HS) et/ou pour les niveaux au-dessous d'un niveau pour une limitation.

7. Procédé selon la revendication 6, dans lequel la variation du diagramme caractéristique d'adaptation du son (KF) pour les niveaux d'entrée est d'au moins 10 dB au-dessus du niveau respectif pour le seuil individuel (HS) et/ou d'au moins 3 dB au-dessous du niveau respectif pour une limitation.

8. Procédé selon l'une des revendications précédentes, dans lequel la reproduction du deuxième signal audio (AS2) a lieu au moyen d'un compresseur multi-bande, où le compresseur multi-bande post-traite en fonction du diagramme caractéristique d'adaptation du son (KF) le deuxième signal audio (AS2) en ce qui concerne les niveaux de sortie dans les différentes plages de fréquences.

9. Procédé selon l'une des revendications précédentes, dans lequel le diagramme caractéristique d'adaptation du son (KF) est choisi de sorte qu'il ne soit pas descendu au-dessous d'un seuil de masquage, où le seuil de masquage est fonction du seuil individuel (HS).

10. Procédé selon l'une des revendications précédentes, dans lequel est ajoutée aux signaux audio, lors de la reproduction de la pluralité de premiers signaux audio (AS1), une part de signal qui permet une simulation des bruits de conduite et/ou de fond.

11. Procédé selon l'une des revendications précédentes, dans lequel le deuxième signal audio (AS2, ASL) est un signal audio à reproduire et l'adaptation du deuxième signal audio (AS2, ASL) est répétée en cours de fonctionnement.

12. Programme d'ordinateur avec un code de programme pour réaliser un procédé selon les revendications 1 à 11 lorsque le programme est exécuté sur un ordinateur.

13. Dispositif de réglage de paramètres pour l'adaptation individuelle d'un signal audio, aux caractéristiques suivantes:
un étage d'adaptation initiale destiné à définir les niveaux pour un seuil individuel (HS) par plage de fréquences des différentes plages de fréquences, avec des moyens destinés à:
reproduire une pluralité de premiers signaux audio (AS1) à différents niveaux et pour différentes plages de fréquences, pour sortir vers un individu une pluralité de premiers signaux acoustiques de différents niveaux de pression acoustique dans les différentes plages de fréquences;
recevoir de l'individu une rétroaction par plage de fréquences des différentes plages de fréquences pour savoir lequel de la pluralité de premiers signaux acoustiques se situe au-dessus du seuil individuel (HS);
dans lequel, par plage de fréquences des différentes plages de fréquences, le niveau le plus bas des différents niveaux de la pluralité de premiers signaux audio pour lequel existe une rétroaction qui caractérise le premier signal audio acoustique associé comme un signal audio acoustique au-dessus du seuil individuel (HS) est utilisé comme niveaux pour les seuils individuels (HS) par plage de fréquences des différentes plages de fréquences;
un autre étage d'adaptation destiné à adapter un deuxième signal audio (AS2), avec des moyens destinés à:
reproduire le deuxième signal audio (AS2) selon un niveau de volume sonore global sélectionné par l'individu, en tenant compte d'un diagramme caractéristique d'adaptation du son (KF), pour sortir vers l'individu un deuxième signal acoustique post-traité; et
faire varier la diagramme caractéristique d'adaptation du son (KF) au moyen d'une interface d'utilisateur ou d'un régulateur jusqu'à ce que l'individu indique par interaction qu'il n'est pas souhaité d'autre variation du diagramme caractéristique d'adaptation du son (KF);
dans lequel le diagramme caractéristique d'adaptation du son (KF) définit une élévation individuelle et/ou un abaissement individuel des niveaux de sortie par niveau d'entrée pour différentes plages de fréquences; dans lequel, dans le diagramme caractéristique d'adaptation du son (KF), les niveaux pour les seuils individuels (HS) par plage de fréquences des différentes plages de fréquences sont utilisés comme niveaux de sortie minimaux.

14. Dispositif selon la revendication 13, dans lequel le dispositif comporte une interface d'utilisateur destinée à recevoir la rétroaction et/ou à faire varier le diagramme caractéristique d'adaptation du son (KF); ou
dans lequel le dispositif comporte une interface d'utilisateur destinée à recevoir la rétroaction et/ou à faire varier le diagramme caractéristique d'adaptation du son (KF) et dans lequel l'interface d'utilisateur comporte un ou plusieurs régulateurs pour l'élévation individuelle et/ou l'abaissement individuel des niveaux de sortie dans au moins deux plages de fréquences ou un champ d'adaptation du son bidimensionnel.

15. Dispositif selon l'une des revendications 13 à 14, dans lequel le dispositif comporte des mémoires destinées à mémoriser le diagramme caractéristique d'adaptation du son (KF).
